# EUROPEAN PATENT APPLICATION

(11) **EP 1 429 139 A1**
(43) Date of publication of application: **16.06.2004**
(21) Application number: 03257764.5
(22) Date of filing: 10.12.2003
(51) Int. Cl.: G01N 27/407

(54) **Gas sensor for SOx measurement**

(30) Priority: 13.12.2002 JP 2002362863
(71) Applicant: Shinko Electric Industries Co., Ltd., Nagano-shi, Nagano 381-2287 (JP)
(72) Inventor: Watanabe, Misa, Shinko Electric Ind. Co., Ltd., Nagano-shi, Nagano 380-0921 (JP); Suganuma, Shigeaki, Shinko Electric Ind. Co., Ltd., Nagano-shi, Nagano 380-0921 (JP); Horiuchi, Michio, Shinko Electric Ind. Co., Ltd., Nagano-shi, Nagano 380-0921 (JP)
(74) Representative: Rackham, Stephen Neil

(57) **Abstract**

A gas sensor (20) for SOx measurement including a substrate (11) comprised of a solid electrolyte material, a sub-electrode (12) comprised of a crystalline sulfate including silver sulfate formed on one surface of the substrate (11), a first electrode (13), including silver formed on the sub-electrode, a second electrode (15) formed on the other surface of the substrate (11), and a gas pipe (22) for introduction of measurement gas covering one surface of the substrate (11), a quartz pipe being used as the gas pipe (22).

## Description

### 1. Field of the Invention

The present invention relates to a gas sensor for measurement of SOx superior in response and able to detect a lower concentration of SOx.

As a gas sensor for measurement of SOx used for a solid electrolyte material, there is known a gas sensor shown in JP-A-7-103937, JP-A-9-80017, etc. FIG. 1 shows the sensor 10 of JP-A-7-103937. Reference numeral 11 is a substrate comprised of yttria-stabilized zirconia ceramic. Reference numeral 12 is a sub electrode formed on one surface of the substrate 11 and comprised of a crystalline sulfate including silver sulfate, 13 is a first electrode including silver formed on the sub electrode 12 (silver-containing electrode), 14 is a protective film comprised of platinum, 15 is a second electrode formed on the other surface of the substrate 11 and comprised of platinum etc., 16 and 17 are lead wires comprised of platinum wire, and 18 is a gas introduction pipe for introduction of measurement gas formed covering one surface of the substrate 11. This gas pipe 18 is formed by yttria-stabilized zirconia ceramic the same as the substrate 11 for matching the heat expansion coefficient with the substrate 11.

By introducing measurement gas into the gas pipe 18, an electrode reaction occurs between the SOx gas and electrode and electromotive force between the first electrode 13 and second electrode 15 is detected, whereby it is possible to compute the amount of gas based on Nernst equation. However, the inventors noticed that the conventional gas sensor for SOx measurement suffered from the following problem. That is, there is known a gas sensor for measurement of COx gas of a similar structure. Compared with this gas sensor for COx measurement, in a gas sensor for SOx measurement, the response time from when the measurement gas is introduced into the gas pipe 18 to when electromotive force is generated is longer. Further, in a conventional gas sensor for SOx measurement, there is the problem that it is only possible to detect a gas content of down to 1 ppm or so.

An object of the present invention is to provide a gas sensor for SOx measurement superior in response and able to detect a lower concentration of SOx.

To achieve the above object, there is provided a gas sensor for SOx measurement comprised of a substrate comprised of a solid electrolyte material, a sub electrode comprised of crystalline sulfate including silver sulfate formed on one surface of the substrate, a first electrode including silver formed on the sub electrode, a second electrode formed on the other surface of the substrate, and a gas pipe for introduction of measurement gas covering one surface of the substrate, a quartz pipe being used as the gas pipe. Preferably, the substrate is comprised of yttria-stabilized zirconia ceramic.

These and other objects and features of the present invention will become clearer from the following description of the preferred embodiments given with reference to the attached drawings, wherein:
FIG. 1 is a sectional view of an example of a gas sensor according to a preferred embodiment of the present invention;
FIG. 2 is a sectional view of another example of a gas sensor according to a preferred embodiment of the present invention;
FIG. 3 is a schematic view of a silver-containing electrode;
FIG. 4 is a sectional view of mesh wire forming the silver-containing electrode of FIG. 3;
FIG. 5 is a graph of the response characteristics of a gas sensor;
FIG. 6 is a graph of a detection limit of SOx gas concentration; and
FIG. 7 is a sectional view of a conventional gas sensor.

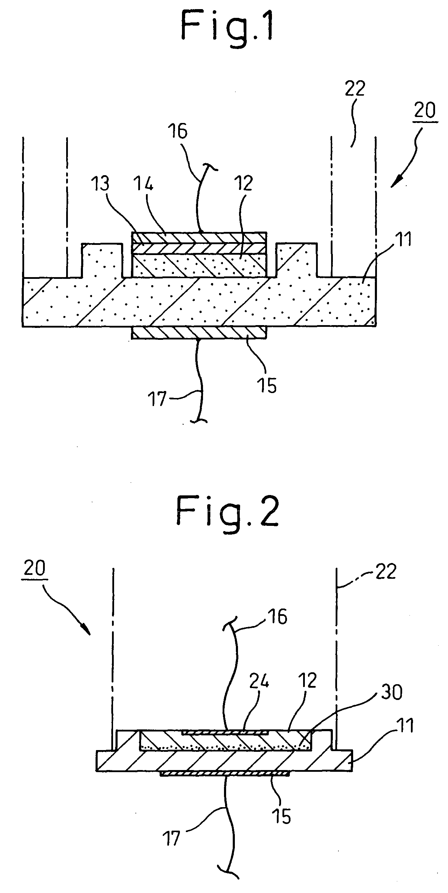

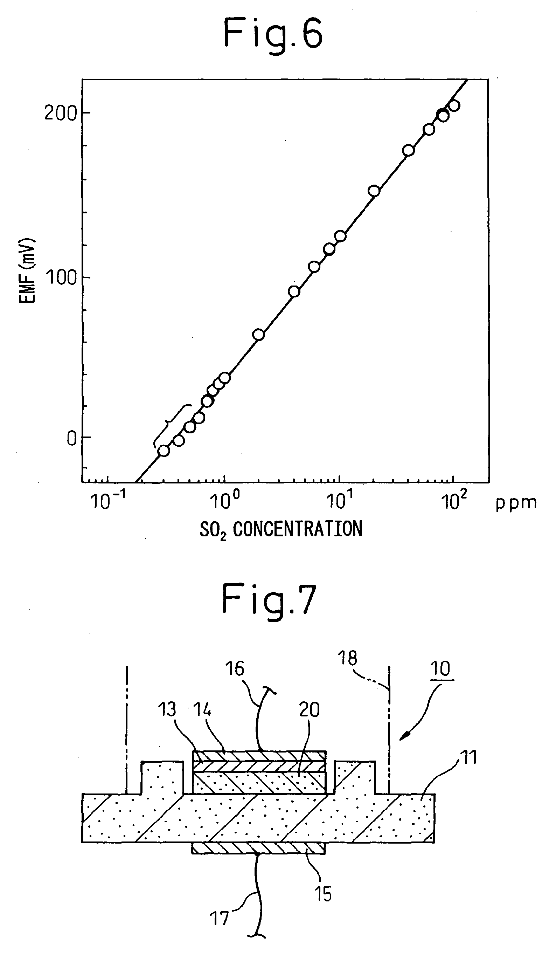

Preferred embodiments of the present invention will be described in detail below while referring to the attached figures. FIG. 1 shows an example of a gas sensor 20. Members the same as those of the prior art shown in FIG. 7 are assigned the same reference numerals. This embodiment is characterized by the use of a quartz pipe as the gas pipe 22. Yttria-stabilized zirconia ceramic is suitably used for the substrate 11. It is possible to air-tightly fix the quartz pipe 22 to the yttria-stabilized zirconia ceramic substrate 11 using sealing glass.

Note that the sub electrode 12 could be made only by silver nitrate, but silver nitrate is expensive and has a low melting point of 654°C, so it is better to use it mixed with another sulfate. As a sulfate for forming the sub electrode, for example, lithium sulfate, barium sulfate, calcium sulfate, or sodium sulfate can be used. Further, by adding these sulfates, it is possible to raise the melting point of the sub electrode as a whole or possible to raise the ion conductivity in the sub electrode and thereby raise the sensitivity of detection of the gas.

Further, metallic silver is used as the metal electrode 13. Note that when making a metal electrode 13 by metallization of metal paste, it is possible to add palladium to the silver to make the electrode. By adding the palladium, it is possible to improve the bondability of the sub electrode 12 and the metal electrode 13. The silver or silver-palladium used for the metal electrode 13 is superior in reactivity with SOx gas. Silver ions are easily produced by the SOx gas. Therefore, it becomes possible to operate the gas sensor even at a relatively low temperature of about 500 to 600°C. Further, the surface of the metal electrode 13 is covered by a platinum film to prevent the metal electrode 13 from being sulfided and to improve the heat resistance when heating the gas sensor to a high temperature. The second electrode 15 may also be formed by platinum.

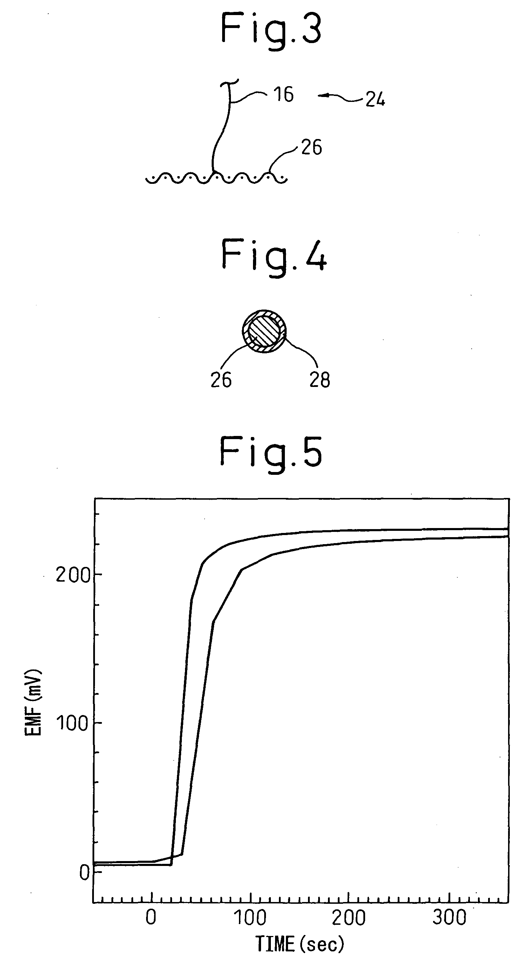

FIG. 2 shows still another embodiment of the gas sensor 20. Members the same as those shown in FIG. 1 are assigned the same reference numerals. In the present embodiment as well, a quartz pipe is used as the gas pipe 22. The sub electrode 12 has formed on its surface a silver-containing electrode 24 serving as the first electrode. This silver-containing electrode 24, as shown in FIG. 3 and FIG. 4, is comprised of a mesh 26 comprised of platinum wire on the surface of which a silver-plating layer 28 is formed. According to this silver-containing electrode 24, it is possible to connect lead wires 16 comprised of platinum wire by welding by spot welds etc. of the mesh 26 and possible to make the connection strength of the two higher.

Further, at the join between the sub electrode 12 and substrate 11, the platinum compound is broken down by heat to form platinum 30. This platinum 30 can be formed by coating for example a hexachloroplatinic acid solution as the platinum compound on the surface of the substrate 11, heating to 300 to 750°C, and breaking down the hexachloroplatinic acid by the heat. By providing platinum at the join in this way, it is possible to shorten the response time of the sensor with respect to changes in concentration of SOx gas. It is guessed that this is because the reaction at the join is promoted by the presence of platinum 30.

In the present invention, as explained above, a quartz pipe was used as the gas pipe 22. If using a yttria-stabilized zirconia ceramic of the same grade as the substrate 11 for the gas pipe 11 as in the prior art, the heat expansion coefficients can be matched, so there is the advantage that cracking etc. can be prevented. However, in the case of a ceramic gas pipe, since ceramic is a sintered body, the inside surface of the gas pipe has a large number of relief shapes on it and therefore the surface area is increased.

The inventors made observations and concluded that particularly in the case of a gas in which sulfur is contained, gas is easily adsorbed at the relief surface and, at the start of introduction of gas, near the inside surface of the gas pipe, gas is adsorbed at the inside surface, the gas adsorption becomes saturated, then gas is discharged and as a result obtained the discovery that the time until reaching the sensor part is lengthened and the time until the electromotive force can be measured, that is, the response time, becomes longer. Based on this discovery, to solve this problem, they conceived of quartz as a material other than ceramic which has heat resistance and has a smooth surface. They tried to form the gas pipe 22 by a quartz pipe, whereupon they found that the actual response time was shortened.

FIG. 5 shows the response characteristics at the time of measurement of SOx gas by the gas sensor 20 of the embodiment of FIG. 1 and FIG. 2 and the conventional sensor 10 shown in FIG. 7. As clear from FIG. 5, the gas sensor 20 according to the present embodiment has both a response start time and startup time shorter than those of the conventional sensor 10. The time until reaching 90% of the rated voltage was about 80 seconds in the case of the conventional sensor, but was halved to about 40 seconds in the case of the present embodiment.

FIG. 6 shows the concentration for detection limit of SOx gas. In the case of the conventional gas sensor, the detection limit was 1 ppm or so, but in the present embodiment, a low concentration of as low as about 0.3 ppm could be detected. In the case of the conventional gas sensor, when the SOx gas is low in concentration, almost all of the gas ended up being adsorbed at the surface of the gas pipe and could not be detected. In the present embodiment, however, the above low concentration of gas could be detected probably because there was almost no adsorption of gas at the inside surface of the gas pipe 22.

Summarizing the effects of the invention, it is able to provide a gas sensor for SOx measurement superior in response and able to detect a lower concentration of SOx.

## Claims

1. A gas sensor for SOx measurement comprised of:
a substrate comprised of a solid electrolyte material,
a sub electrode comprised of crystalline sulfate including silver sulfate formed on one surface of the substrate,
a first electrode including silver formed on said sub electrode,
a second electrode formed on the other surface of the substrate, and
a gas pipe for introduction of measurement gas covering one surface of said substrate,
a quartz pipe being used as said gas pipe.

2. A gas sensor for SOx measurement as set forth in claim 1, wherein said substrate is comprised of yttria-stabilized zirconia ceramic.
